## Europäisches Patentamt
## European Patent Office
## Office européen des brevets

(19)

(11) Publication number: **0 016 487**
**B1**

(12) # EUROPEAN PATENT SPECIFICATION

(45) Date of publication of patent specification: **01.06.83**

(21) Application number: **80200175.0**

(22) Date of filing: **29.02.80**

(51) Int. Cl.³: **C 07 C 47/54,**
**C 07 C 45/85, C 07 C 45/82**

(54) **Method for the purification of benzaldehyde.**

(30) Priority: **02.03.79 NL 7901669**

(43) Date of publication of application:
**01.10.80 Bulletin 80/20**

(45) Publication of the grant of the patent:
**01.06.83 Bulletin 83/22**

(84) Designated Contracting States:
**BE DE FR GB IT NL**

(56) References cited:
**NL - A - 275 790**

(73) Proprietor: **STAMICARBON B.V.**
**Postbus 10**
**NL-6160 MC Geleen (NL)**

(72) Inventor: **Jongsma, Cornelis**
**Drossaertweide 1**
**NL-6438 HS Oirsbeek (NL)**

(74) Representative: **Hoogstraten, Willem Cornelis**
**Roeland et al,**
**OCTROOIBUREAU DSM Postbus 9**
**NL-6160 MA Geleen (NL)**

Courier Press, Leamington Spa, England

# Method for the purification of benzaldehyde

The invention relates to a method for the purification of benzaldehyde, in particular of benzaldehyde prepared by oxidation of toluene with a gas containing molecular oxygen.

Benzaldehyde is an important starting material in chemical synthesis, for instance in the synthesis of flavours and fragrances. For such applications, the benzaldehyde is often required to have a high degree of purity. However, crude benzaldehyde, in particular benzaldehyde prepared by oxidation of toluene with a gas containing molecular oxygen, contains troublesome impurities, which are very difficult to remove. It is pointed out that benzylhydroperoxide normally is not present in significant quantities in the impure benzaldehyde. In particular, it is very difficult to prepare from the crude benzaldehyde a product which meets olfactory specifications. The presence of impurities manifests itself in the rather early discoloration of stored benzaldehyde, occurring even at a very low concentration (ppm by wt. level) of the impurities.

It has been proposed (Japanese patent publication No. 24.467/74) to purify benzal-,dehyde by treating it with an aqueous sodium hydroxide solution. This purification method does not give satisfactory results, however. It appears that the treated benaldehyde still discolours rather soon.

It is known from the Dutch application NL—A—275 790 to stabilize ketones against changes in colour and/or odour during storage by treating the ketone with hydrogen in the presence of a hydrogenation catalyst. This process is described especially for aliphatic ketones having three to ten carbon atoms per molecule, particularly those which are saturated or, if unsaturated, have not more than one pair of multiple-bonded carbon atoms per substituted or unsubstituted hydrocarbon radical (page 1, lines 1—8). However since benzaldehyde is not a ketone nor an aliphatic compound, has more than one pair of multiple-bonded carbon atoms in the hydrocarbon radical and contains other impurities to be removed because of the different method of preparation, the stabilization according to the Dutch application NL—A—275 790 is considered to be scarcely relevant to the stabilisation of benzaldehyde.

The objective of the invention is to provide a solution to said problem. According to the invention, pure benzaldehyde is obtained by treating impure benzaldehyde, containing no significant quantities of benzylhydroperoxide, with hydrogen in the presence of a suitable hydrogenation catalyst for the purpose of hydrogenation of impurities without significant hydrogenation of benzaldehyde and next distilling it.

In the treatment according to the invention the loss of benzaldehyde is very small and may be in the order of 1—5% wt. Benzaldehyde meeting olfactory specifications may be obtained even from crude benzaldehyde prepared by oxidation of toluene.

Suitable hydrogenation catalysts for the method according to the invention are the known hydrogenation catalyst, for instance those on the basis of metals of group VIII of the periodic system, such as palladium, nickel, platinum, iridium or rhodium. The catalytic material may be on a carrier, for instance carbon, aluminium oxide, silicon or titanium oxide. Particularly suitable catalysts for this method are Raney nickel, and palladium on carbon.

The catalysts are preferably used in quantities of 0.5—200 mg at active substance per kg benzaldehyde. Particularly suitable are quantities of 1—100 mg at active substance per kg benzaldehyde. In the method according to the invention, 1—100 litres hydrogen (N.T.P.) per kg benzaldehyde per hour is normally taken up. Often 3—40 litres hydrogen (N.T.P.) per kg benzaldehyde is taken up. The contacting of the benzaldehyde with the hydrogen may be effected in various manners. For instance, the benzaldehyde may be stirred in a hydrogen atmosphere for a while or the hydrogen may be bubbled through or passed over the benzaldehyde. The duration of the hydrogenation treatment according to the invention usually is between 0.25 and 4 hours, preferably between 0.5 and 2 hours. The use of larger quantities of catalyst or of large excess of hydrogen, and/or extension of the treatment time is/are not excluded, but offer(s) no advantages.

Treatment of impure benzaldehyde with hydrogen in the presence of suitable hydrogenation catalyst is preferably effected at a moderate temperature in order to suppress hydrogenation of benzaldehyde. A suitable temperature range is between 270 and 400 K. Particularly suitable are temperatures between 285 and 340 K. The reaction pressure should be such that the liquid phase is maintained. A suitable reaction pressure is, for instance, between 100 and 1000 kPa, in particular between 100 and 500 kPa. The distillation subsequent to the hydrogenation treatment may be carried out at atmospheric or elevated pressure, but preferably at reduced pressure, for instance a pressure of 2—35 kPa.

It is pointed out that it is known from the US patent specification No. 3.387.036 that an oxidation product of toluene, consisting of a mixture of toluene, benzylhydroperoxide, benzaldehyde, benzoic acid and some other byproducts, may be processed in such a manner that the benzylhydroperoxide is converted, for instance by catalytic hydrogenation of this compound. However, such a 'de-peroxidation' is completely different from the method according to the invention.

The invention will be elucidated by means of the following non-restrictive examples and comparative experiment. The colour value in degrees Hazen (°H) was determined by ASTM D 1209/62.

Example I

A sample of benzaldehyde, prepared by oxidation of toluene in the liquid phase by means of a gas containing molecular oxygen with the use of a homogeneous cobalt catalyst, was treated with hydrogen for 2 hours at 295 K and a pressure of 350 kPa in the presence of 0.5% by wt. Raney nickel, during which treatment 10 litres hydrogen (N.T.P.) per kg benzaldehyde per hour was taken up. Subsequently, the mixture was distilled in a sieve-tray column with 30 trays at a top pressure of 20 kPa and with a reflux ratio of 1:3. The colour value of the main fraction was 10°H. This main fraction was divided into two portions. One portion was heated for 1 hour under a nitrogen atmosphere. The colour value rose to 25°H. The other portion was stored for 30 days in a dark bottle under a nitrogen atmosphere. At the end of the period, the colour value had risen to 20°H.

Example II

A sample of the same liquid crude benzaldehyde as used in example I was treated with hydrogen for 0.5 hour at 305 K and a pressure of 300 kPa in the presence of 0.5% by wt. 5% palladium-on-carbon catalyst, during which treatment 30 litres hydrogen (N.T.P.) per kg benzaldehyde per hour was taken up.

Subsequently, the mixture was distilled under the same circumstances as in example I. The colour value of the main fraction was 10°H. This main fraction was divided into two portions.

One portion was heated for 1 hour under a nitrogen atmosphere. The colour value rose to 35°H. The other portion was stored for 30 days in a dark bottle under a nitrogen atmosphere. At the end of this period, the colour value had risen to 25°H.

Comparative Experiment

A sample of the same liquid benzaldehyde as used in example I was distilled without previous treatment, under the same circumstances as in example I. The colour value of the main fraction was 25°H. This main fraction was divided into two portions. One portion was heated under a nitrogen atmosphere. After 0.2 hour, the colour value of this portion had already risen to well over 100°H. The other portion was stored for 30 days in a dark bottle under a nitrogen atmosphere. At the end of this period, the colour value had risen to 50°H.

**Claims**

1. Method for the preparation of pure benzaldehyde, characterized in that impure benzaldehyde, containing no significant quantities of benzylhydroperoxide, is treated with hydrogen in the presence of a suitable hydrogenation catalyst in order to hydrogenate impurities without essential hydrogenation of benzaldehyde, and is next distilled.

2. Method according to claim 1, characterized in that the impure benzaldehyde was obtained by oxidation of toluene by means of a gas containing molecular oxygen.

3. Method according to claim 1 or 2, characterized in that the catalyst used contains a metal of group VIII of the periodic system.

4. Method according to claim 3, characterized in that Raney nickel is used as hydrogenation catalyst.

5. Method according to claim 3, characterized in that palladium-on-carbon is used as hydrogenation catalyst.

6. Method according to any one of the claims 1—5, characterized in that the hydrogenation catalyst is used in a quantity 0.5—200 mg at active substance per kg benzaldehyde.

7. Method according to claim 6, characterized in that the hydrogenation catalyst is used in a quantity of 1—100 mg at active substance per kg benzaldehyde.

8. Method according to any one of the claims 1—7, characterized in that the duration of the hydrogen treatment is between 0.25 and 4 hours.

9. Method according to any one of the claims 1—8, characterized in that the hydrogenation treatment is effected at a temperature between 270 and 400 K.

10. Method according to any one of the claims 1—9, characterized in that the hydrogenation treatment is effected at a pressure of 100—1000 kPa.

**Revendications**

1. Procédé de préparation de benzaldéhyde pur, caractérisé en ce que du benzaldéhyde impur, qui ne contient pas de quantités importantes d'hydroperoxyde de benzyle, est traité avec de l'hydrogène en présence d'un catalyseur d'hydrogénation approprié afin d'hydrogéner les impuretés sans hydrogénation importante du benzaldéhyde et qu'il est distillé ensuite.

2. Procédé selon la revendication 1, caractérisé en ce qu'on obtient le benzaldéhyde impur par l'oxydation du toluène à l'aide d'un gaz contenant de l'oxygène moléculaire.

3. Procédé selon l'une des revendications 1 et 2, caractérisé en ce que le catalyseur utilisé contient un métal du groupe VIII du système périodique.

4. Procédé selon la revendication 3, caractérisé en ce qu'on utilise du nickel de Raney comme catalyseur d'hydrogénation.

5. Procédé selon la revendication 3, caractérisé en ce qu'on utilise du palladium-sur-carbone comme catalyseur d'hydrogénation.

6. Procédé selon l'une des revendications 1 à 5, caractérisé en ce que le catalyseur d'hydrogénation est utilisé dans une quantité de 0,5—200 mg de substance active par kg de benzaldéhyde.

7. Procédé selon la revendication 6, caractérisé en ce que le catalyseur d'hydrogénation est utilisé dans une quantité de 1—100 mg de substance active par kg de benzaldéhyde.

8. Procédé selon l'une des revendications 1 à 7, caractérisé en ce que la durée de l'hydrogénation se situe entre 0,25 et 4 heures.

9. Procédé selon l'une des revendications 1 à 8, caractérisé en ce que l'hydrogénation est exécutée à une température située entre 270 et 400 K.

10. Procédé selon l'une des revendications 1 à 9, caractérisé en ce que l'hydrogénation est exécutée à une pression de 100—1000 kPa.

## Patentansprüche

1. Verfahren zur Herstellung von reinem Benzaldehyd, dadurch gekennzeichnet, dass unreines Benzaldehyd, welches keine signifikanten Mengen von Benzylhydroperoxid enthält, mit Wasserstoff in Anwesenheit eines geeigneten Hydrierkatalysators behandelt wird, um Fremdstoffe ohne wesentliche Hydrierung von Benzaldehyd zu hydrieren, und anschliessend destilliert wird.

2. Verfahren nach Anspruch 1, dadurch gekennzeichnet, dass das unreine Benzaldehyd durch Oxydation von Toluol mittels eines molekularen Sauerstoff enthaltenden Gases erhalten wurde.

3. Verfahren nach Anspruch 1 oder 2, dadurch gekennzeichnet, dass der verwendete Katalysator ein Metall der Gruppe VIII des Periodensystems enthält.

4. Verfahren nach Anspruch 3, dadurch gekennzeichnet, dass Raney-Nickel als Hydrierkatalysator verwendet wird.

5. Verfahren nach Anspruch 3, dadurch gekennzeichnet, dass Palladium-auf-Kohlenstoff als Hydrierkatalysator verwendet wird.

6. Verfahren nach einem der Ansprüche 1—5, dadurch gekennzeichnet, dass der hydrierkatalysator in einer Menge von 0,5—200 mg in der aktiven Substanz je kg Benzaldehyd verwendet wird.

7. Verfahren nach Anspruch 6, dadurch gekennzeichnet, dass der Hydrierkatalysator in einer Menge von 1—100 mg in der aktiven Substanz je kg Benzaldehyd verwendet wird.

8. Verfahren nach einem der Ansprüche 1—7, dadurch gekennzeichnet, dass die Dauer der Hydrierbehandlung zwischen 0,25 und 4 stunden beträgt.

9. Verfahren nach einem der Ansprüche 1—8, dadurch gekennzeichnet, dass die Hydrierbehandlung bei einer Temperatur zwischen 270 und 400 K durchgeführt wird.

10. Verfahren nach einem der Ansprüche 1—9, dadurch gekennzeichnet, dass die Hydrierbehandlung bei einem Druck von 100—1000 kPa durchgeführt wird.